# EUROPEAN PATENT APPLICATION

(11) **EP 4 692 120 A1**
(43) Date of publication of application: **11.02.2026**
(21) Application number: 24778150.3
(22) Date of filing: 28.03.2024
(51) Int. Cl.: C07K 16/28, C12N 15/13, A61K 39/395, A61P 35/00, A61P 7/06

(54) **ANTI-LAG3 ANTIBODY, PHARMACEUTICAL COMPOSITION, AND USE**

(30) Priority: 29.03.2023 CN 202310327266
(71) Applicant: Akeso Biopharma Co., Ltd., Zhongshan, Guangdong 528437 (CN)
(72) Inventor: LI, Baiyong, Zhongshan, Guangdong 528437 (CN); XIA, Yu, Zhongshan, Guangdong 528437 (CN)
(74) Representative: Santarelli
(86) International application number: PCT/CN2024/084453
(87) International publication number: WO 2024/199364

(57) **Abstract**

The present invention relates to an anti-LAG3 antibody, a pharmaceutical composition, and a use. Specifically, the present invention relates to an anti-LAG3 antibody or an antigen-binding fragment thereof. The anti-LAG3 antibody comprises a heavy chain variable region and a light chain variable region. The heavy chain variable region comprises HCDR1, HCDR2 and HCDR3, and the light chain variable region comprises LCDR1, LCDR2 and LCDR3. The amino acid sequence of HCDR1 is as shown in SEQ ID NO: 9, the amino acid sequence of HCDR2 is as shown SEQ ID NO: 10, and the amino acid sequence of HCDR3 is as shown in SEQ ID NO: 11. The amino acid sequence of LCDR1 is as shown in SEQ ID NO: 12 or SEQ ID NO: 17, the amino acid sequence of LCDR2 is as shown in SEQ ID NO: 13 or SEQ ID NO: 15, and the amino acid sequence of LCDR3 is as shown in SEQ ID NO: 14 or SEQ ID NO: 16. The anti-LAG3 antibody has excellent affinity and specificity, and has good prospects for anti-tumor application.

## Description

### CROSS-REFERENCE TO RELATED APPLICATIONS

The present application is based on and claims priority to Chinese Patent Application No. CN202310327266.6 filed on Mar. 29, 2023, which is incorporated herein by reference in its entirety.

### TECHNICAL FIELD

The present disclosure pertains to the field of biomedicine and relates to an anti-LAG3 antibody, a pharmaceutical composition comprising an anti-LAG3 antibody or an antigen-binding fragment thereof, and use.

### BACKGROUND

Tumor, especially a malignant tumor, is a serious health-threatening disease in the world today, and it is the second leading cause of death among various diseases. In recent years, the incidence of the disease has been increasing remarkably. The malignant tumor is characterized by poor therapeutic effect, high late metastasis rate, and poor prognosis. Although conventional treatment methods (such as radiotherapy, chemotherapy, and surgical treatment) adopted clinically at present alleviate the pain to a great extent and prolong the survival time, the methods have great limitations, and it is difficult to further improve their efficacy.

Lymphocyte-activation gene 3 (LAG3), namely CD223, is a type I transmembrane protein composed of 498 amino acids and is a member of the immunoglobulin superfamily (IgSF). LAG3 is mainly expressed in activated CD4⁺ T cells and CD8⁺ T cells. Additionally, in cells such as natural killer (NK) cells, B cells, regulatory T cells (Tregs), and plasmacytoid dendritic cells (pDCs), LAG3 is also expressed. (Ruffo Elisa,Wu Richard C,Bruno Tullia C et al. Lymphocyte-activation gene 3 (LAG3): The next immune checkpoint receptor.[J]. Semin Immunol, 2019, 42: 101305).

The LAG3 molecule gene is located on human chromosome 12 (12p13.3), adjacent to the CD4 molecule gene, and both have the same exons and introns. The LAG3 molecule and the CD4 molecule have a high degree of structural similarity, although the amino acid sequence homology between the two is only about 20%. Major histocompatibility complex class II (MHC-II) molecules, liver sinusoidal endothelial cell lectin (LSECtin) molecules, and galectin-3 molecules are related ligands for the LAG3 molecule. The MHC-II molecules are the main ligands for LAG3. The affinity (Kd: 60 nmol·L⁻¹) of LAG3 molecules for the MHC-II molecules is 100 times that of CD4 molecules, indicating that the LAG3 molecules can effectively compete with the CD4 molecules for binding to the MHC-II molecules and inhibit T cell activation.

In the tumor microenvironment, the expression of the immunosuppressive molecule LAG3 can be detected 24 hours after T cell activation, which then leads to T cell dysfunction or apoptosis. The LAG3 molecule, through its D1 domain (which contains one proline-rich loop structure), forms a dimer molecule to specifically bind to the MHC-II molecule in the first signaling axis of CD4⁺ T cell activation, "CD3-TCR-MHC-II", so that in one aspect, a signal transduction pathway for T cell activation is blocked, and in another aspect, an intracellular segment of the LAG3 molecule (KIEELE motif) generates an immunosuppressive signal to down-regulate the activity of CD4⁺ T cells. The LAG3 molecule can promote the differentiation of Treg cells, participate in downstream signaling of signal transducer and activator of transcription 5, and thus enhance the inhibitory effect of Treg cells. This is one of the mechanisms by which tumors escape from killing by the immune system (Andrews Lawrence P, Marciscano Ariel E, Drake Charles G, et al., LAG3 (CD223) as a cancer immunotherapy target. [J]. Immunol Rev, 2017, 276: 80-96).

Multiple studies have shown that LAG3 is overexpressed in tumor-infiltrating CD8⁺ T cells of various malignant tumors. For example, in ovarian cancer, tumor-infiltrating New York esophageal squamous cell carcinoma 1 (NY-ESO-1) antigen-specific CD8⁺ T cells express high levels of PD-1 and LAG3, and have a reduced ability to produce IFN-γ and TNF-α, thereby leading to lymphocyte inactivation. Galectin-3 and LSECtin interact primarily with LAG3 to regulate the activation and function of CD8⁺ T cells. In addition, melanoma antigen-specific T cells isolated from patients with metastatic melanoma exhibit a significant up-regulation in the expression of LAG3 and other immune checkpoint molecules CTLA-4 and TIM-3. (Liu Hao, Li Xinying, Luo Longlong, et al., Research advances in biological function of lymphocyte activation gene-3 (LAG-3) molecule and clinical application of antibody drugs targeting LAG-3 [J]. Chinese Journal of Pharmacology and Toxicology, 2019, 33(01): 70-78).

Currently, several LAG3 antibody drugs have entered the clinical research stages, among which Bristol Myers Squibb's Relatlimab has progressed the fastest, with 10 clinical studies underway. The vast majority of these studies involve the combination therapy of Relatlimab with nivolumab for the treatment of tumors such as hematological malignancies, melanoma, glioblastoma, renal cell carcinoma, non-small cell lung cancer, and the like.

Currently, there is a need to develop a novel anti-LAG3 antibody drug.

### SUMMARY

Through intensive studies and creative efforts, the inventors have obtained an anti-LAG3 antibody. The inventors have surprisingly found that the anti-LAG3 antibody of the present disclosure (also referred to as the antibody or the antibody of the present disclosure for short) has superior affinity and/or specificity, and is comparable to or even better than that of the positive control antibody (such as Relatlimab) in one or more aspects. The present disclosure is detailed below:
One aspect of the present disclosure relates to an anti-LAG3 antibody or an antigen-binding fragment thereof, wherein the anti-LAG3 antibody comprises a heavy chain variable region and a light chain variable region, the heavy chain variable region comprises HCDR1, HCDR2, and HCDR3, and the light chain variable region comprises LCDR1, LCDR2, and LCDR3, wherein
HCDR1 comprises an amino acid sequence set forth in SEQ ID NO: 9, HCDR2 comprises an amino acid sequence set forth in SEQ ID NO: 10, and
HCDR3 comprises an amino acid sequence set forth in SEQ ID NO: 11; LCDR1 comprises an amino acid sequence set forth in SEQ ID NO: 12 or SEQ ID NO: 17,
LCDR2 comprises an amino acid sequence set forth in SEQ ID NO: 13 or SEQ ID NO: 15, and
LCDR3 comprises an amino acid sequence set forth in SEQ ID NO: 14 or SEQ ID NO: 16.

In some embodiments of the present disclosure, provided is the anti-LAG3 antibody or the antigen-binding fragment thereof, wherein,
the amino acid sequence of HCDR1 is set forth in SEQ ID NO: 9,
the amino acid sequence of HCDR2 is set forth in SEQ ID NO: 10,
the amino acid sequence of HCDR3 is set forth in SEQ ID NO: 11;
the amino acid sequence of LCDR1 is set forth in SEQ ID NO: 12 or SEQ ID NO: 17,
the amino acid sequence of LCDR2 is set forth in SEQ ID NO: 13 or SEQ ID NO: 15,
the amino acid sequence of LCDR3 is set forth in SEQ ID NO: 14 or SEQ ID NO: 16.

In some embodiments of the present disclosure, provided is the anti-LAG3 antibody or the antigen-binding fragment thereof, wherein,
LCDR1 comprises an amino acid sequence set forth in SEQ ID NO: 12,
LCDR2 comprises an amino acid sequence set forth in SEQ ID NO: 13, and
LCDR3 comprises an amino acid sequence set forth in SEQ ID NO: 14; or
LCDR1 comprises an amino acid sequence set forth in SEQ ID NO: 12,
LCDR2 comprises an amino acid sequence set forth in SEQ ID NO: 15, and
LCDR3 comprises an amino acid sequence set forth in SEQ ID NO: 16; or
LCDR1 comprises an amino acid sequence set forth in SEQ ID NO: 17,
LCDR2 comprises an amino acid sequence set forth in SEQ ID NO: 15, and
LCDR3 comprises an amino acid sequence set forth in SEQ ID NO: 14; or
LCDR1 comprises an amino acid sequence set forth in SEQ ID NO: 12,
LCDR2 comprises an amino acid sequence set forth in SEQ ID NO: 13, and
LCDR3 comprises an amino acid sequence set forth in SEQ ID NO: 16; or
LCDR1 comprises an amino acid sequence set forth in SEQ ID NO: 12,
LCDR2 comprises an amino acid sequence set forth in SEQ ID NO: 15, and
LCDR3 comprises an amino acid sequence set forth in SEQ ID NO: 14; or
LCDR1 comprises an amino acid sequence set forth in SEQ ID NO: 17,
LCDR2 comprises an amino acid sequence set forth in SEQ ID NO: 13, and
LCDR3 comprises an amino acid sequence set forth in SEQ ID NO: 14; or
LCDR1 comprises an amino acid sequence set forth in SEQ ID NO: 17,
LCDR2 comprises an amino acid sequence set forth in SEQ ID NO: 13, and
LCDR3 comprises an amino acid sequence set forth in SEQ ID NO: 16; or
LCDR1 comprises an amino acid sequence set forth in SEQ ID NO: 17,
LCDR2 comprises an amino acid sequence set forth in SEQ ID NO: 15, and
LCDR3 comprises an amino acid sequence set forth in SEQ ID NO: 16.

In some embodiments of the present disclosure, provided is the anti-LAG3 antibody or the antigen-binding fragment thereof, wherein,
the amino acid sequence of LCDR1 is set forth in SEQ ID NO: 12,
the amino acid sequence of LCDR2 is set forth in SEQ ID NO: 13, and
the amino acid sequence of LCDR3 is set forth in SEQ ID NO: 14; or
the amino acid sequence of LCDR1 is set forth in SEQ ID NO: 12,
the amino acid sequence of LCDR2 is set forth in SEQ ID NO: 15, and
the amino acid sequence of LCDR3 is set forth in SEQ ID NO: 16; or
the amino acid sequence of LCDR1 is set forth in SEQ ID NO: 17,
the amino acid sequence of LCDR2 is set forth in SEQ ID NO: 15, and
the amino acid sequence of LCDR3 is set forth in SEQ ID NO: 14; or
the amino acid sequence of LCDR1 is set forth in SEQ ID NO: 12,
the amino acid sequence of LCDR2 is set forth in SEQ ID NO: 13, and
the amino acid sequence of LCDR3 is set forth in SEQ ID NO: 16; or
the amino acid sequence of LCDR1 is set forth in SEQ ID NO: 12,
the amino acid sequence of LCDR2 is set forth in SEQ ID NO: 15, and
the amino acid sequence of LCDR3 is set forth in SEQ ID NO: 14; or
the amino acid sequence of LCDR1 is set forth in SEQ ID NO: 17,
the amino acid sequence of LCDR2 is set forth in SEQ ID NO: 13, and
the amino acid sequence of LCDR3 is set forth in SEQ ID NO: 14; or
the amino acid sequence of LCDR1 is set forth in SEQ ID NO: 17,
the amino acid sequence of LCDR2 is set forth in SEQ ID NO: 13, and
the amino acid sequence of LCDR3 is set forth in SEQ ID NO: 16; or
the amino acid sequence of LCDR1 is set forth in SEQ ID NO: 17,
the amino acid sequence of LCDR2 is set forth in SEQ ID NO: 15, and
the amino acid sequence of LCDR3 is set forth in SEQ ID NO: 16.

In some embodiments of the present disclosure, provided is the anti-LAG3 antibody or the antigen-binding fragment thereof, wherein,
HCDR1 comprises an amino acid sequence set forth in SEQ ID NO: 9,
HCDR2 comprises an amino acid sequence set forth in SEQ ID NO: 10,
HCDR3 comprises an amino acid sequence set forth in SEQ ID NO: 11;
LCDR1 comprises an amino acid sequence set forth in SEQ ID NO: 12,
LCDR2 comprises an amino acid sequence set forth in SEQ ID NO: 13, and
LCDR3 comprises an amino acid sequence set forth in SEQ ID NO: 14.

In some embodiments of the present disclosure, provided is the anti-LAG3 antibody or the antigen-binding fragment thereof, wherein,
HCDR1 comprises an amino acid sequence set forth in SEQ ID NO: 9,
HCDR2 comprises an amino acid sequence set forth in SEQ ID NO: 10,
HCDR3 comprises an amino acid sequence set forth in SEQ ID NO: 11;
LCDR1 comprises an amino acid sequence set forth in SEQ ID NO: 12,
LCDR2 comprises an amino acid sequence set forth in SEQ ID NO: 15, and
LCDR3 comprises an amino acid sequence set forth in SEQ ID NO: 16.

In some embodiments of the present disclosure, provided is the anti-LAG3 antibody or the antigen-binding fragment thereof, wherein,
HCDR1 comprises an amino acid sequence set forth in SEQ ID NO: 9,
HCDR2 comprises an amino acid sequence set forth in SEQ ID NO: 10,
HCDR3 comprises an amino acid sequence set forth in SEQ ID NO: 11;
LCDR1 comprises an amino acid sequence set forth in SEQ ID NO: 17,
LCDR2 comprises an amino acid sequence set forth in SEQ ID NO: 15, and
LCDR3 comprises an amino acid sequence set forth in SEQ ID NO: 14.

In some embodiments of the present disclosure, provided is the anti-LAG3 antibody or the antigen-binding fragment thereof, wherein,
HCDR1 comprises an amino acid sequence set forth in SEQ ID NO: 9,
HCDR2 comprises an amino acid sequence set forth in SEQ ID NO: 10,
HCDR3 comprises an amino acid sequence set forth in SEQ ID NO: 11;
LCDR1 comprises an amino acid sequence set forth in SEQ ID NO: 12,
LCDR2 comprises an amino acid sequence set forth in SEQ ID NO: 13, and
LCDR3 comprises an amino acid sequence set forth in SEQ ID NO: 16.

In some embodiments of the present disclosure, provided is the anti-LAG3 antibody or the antigen-binding fragment thereof, wherein,
HCDR1 comprises an amino acid sequence set forth in SEQ ID NO: 9,
HCDR2 comprises an amino acid sequence set forth in SEQ ID NO: 10,
HCDR3 comprises an amino acid sequence set forth in SEQ ID NO: 11;
LCDR1 comprises an amino acid sequence set forth in SEQ ID NO: 12,
LCDR2 comprises an amino acid sequence set forth in SEQ ID NO: 15, and
LCDR3 comprises an amino acid sequence set forth in SEQ ID NO: 14.

In some embodiments of the present disclosure, provided is the anti-LAG3 antibody or the antigen-binding fragment thereof, wherein,
HCDR1 comprises an amino acid sequence set forth in SEQ ID NO: 9,
HCDR2 comprises an amino acid sequence set forth in SEQ ID NO: 10,
HCDR3 comprises an amino acid sequence set forth in SEQ ID NO: 11;
LCDR1 comprises an amino acid sequence set forth in SEQ ID NO: 17,
LCDR2 comprises an amino acid sequence set forth in SEQ ID NO: 13, and
LCDR3 comprises an amino acid sequence set forth in SEQ ID NO: 14.

In some embodiments of the present disclosure, provided is the anti-LAG3 antibody or the antigen-binding fragment thereof, wherein,
HCDR1 comprises an amino acid sequence set forth in SEQ ID NO: 9,
HCDR2 comprises an amino acid sequence set forth in SEQ ID NO: 10,
HCDR3 comprises an amino acid sequence set forth in SEQ ID NO: 11;
LCDR1 comprises an amino acid sequence set forth in SEQ ID NO: 17,
LCDR2 comprises an amino acid sequence set forth in SEQ ID NO: 13, and
LCDR3 comprises an amino acid sequence set forth in SEQ ID NO: 16.

In some embodiments of the present disclosure, provided is the anti-LAG3 antibody or an antigen-binding fragment thereof, wherein
HCDR1 comprises an amino acid sequence set forth in SEQ ID NO: 9,
HCDR2 comprises an amino acid sequence set forth in SEQ ID NO: 10,
HCDR3 comprises an amino acid sequence set forth in SEQ ID NO: 11;
LCDR1 comprises an amino acid sequence set forth in SEQ ID NO: 17,
LCDR2 comprises an amino acid sequence set forth in SEQ ID NO: 15, and
LCDR3 comprises an amino acid sequence set forth in SEQ ID NO: 16.

In some embodiments of the present disclosure, provided is the anti-LAG3 antibody or the antigen-binding fragment thereof, wherein
the amino acid sequence of HCDR1 is set forth in SEQ ID NO: 9,
the amino acid sequence of HCDR2 is set forth in SEQ ID NO: 10,
the amino acid sequence of HCDR3 is set forth in SEQ ID NO: 11;
the amino acid sequence of LCDR1 is set forth in SEQ ID NO: 12,
the amino acid sequence of LCDR2 is set forth in SEQ ID NO: 13, and
the amino acid sequence of LCDR3 is set forth in SEQ ID NO: 14.

In some embodiments of the present disclosure, provided is the anti-LAG3 antibody or the antigen-binding fragment thereof, wherein
the amino acid sequence of HCDR1 is set forth in SEQ ID NO: 9,
the amino acid sequence of HCDR2 is set forth in SEQ ID NO: 10,
the amino acid sequence of HCDR3 is set forth in SEQ ID NO: 11;
the amino acid sequence of LCDR1 is set forth in SEQ ID NO: 12,
the amino acid sequence of LCDR2 is set forth in SEQ ID NO: 15, and
the amino acid sequence of LCDR3 is set forth in SEQ ID NO: 16.

In some embodiments of the present disclosure, provided is the anti-LAG3 antibody or the antigen-binding fragment thereof, wherein
the amino acid sequence of HCDR1 is set forth in SEQ ID NO: 9,
the amino acid sequence of HCDR2 is set forth in SEQ ID NO: 10,
the amino acid sequence of HCDR3 is set forth in SEQ ID NO: 11;
the amino acid sequence of LCDR1 is set forth in SEQ ID NO: 17,
the amino acid sequence of LCDR2 is set forth in SEQ ID NO: 15, and
the amino acid sequence of LCDR3 is set forth in SEQ ID NO: 14.

In some embodiments of the present disclosure, provided is the anti-LAG3 antibody or the antigen-binding fragment thereof, wherein
the amino acid sequence of HCDR1 is set forth in SEQ ID NO: 9,
the amino acid sequence of HCDR2 is set forth in SEQ ID NO: 10,
the amino acid sequence of HCDR3 is set forth in SEQ ID NO: 11;
the amino acid sequence of LCDR1 is set forth in SEQ ID NO: 12,
the amino acid sequence of LCDR2 is set forth in SEQ ID NO: 13, and
the amino acid sequence of LCDR3 is set forth in SEQ ID NO: 16.

In some embodiments of the present disclosure, provided is the anti-LAG3 antibody or the antigen-binding fragment thereof, wherein
the amino acid sequence of HCDR1 is set forth in SEQ ID NO: 9,
the amino acid sequence of HCDR2 is set forth in SEQ ID NO: 10,
the amino acid sequence of HCDR3 is set forth in SEQ ID NO: 11;
the amino acid sequence of LCDR1 is set forth in SEQ ID NO: 12,
the amino acid sequence of LCDR2 is set forth in SEQ ID NO: 15, and
the amino acid sequence of LCDR3 is set forth in SEQ ID NO: 14.

In some embodiments of the present disclosure, provided is the anti-LAG3 antibody or the antigen-binding fragment thereof, wherein
the amino acid sequence of HCDR1 is set forth in SEQ ID NO: 9,
the amino acid sequence of HCDR2 is set forth in SEQ ID NO: 10,
the amino acid sequence of HCDR3 is set forth in SEQ ID NO: 11;
the amino acid sequence of LCDR1 is set forth in SEQ ID NO: 17,
the amino acid sequence of LCDR2 is set forth in SEQ ID NO: 13, and
the amino acid sequence of LCDR3 is set forth in SEQ ID NO: 14.

In some embodiments of the present disclosure, provided is the anti-LAG3 antibody or the antigen-binding fragment thereof, wherein
the amino acid sequence of HCDR1 is set forth in SEQ ID NO: 9,
the amino acid sequence of HCDR2 is set forth in SEQ ID NO: 10,
the amino acid sequence of HCDR3 is set forth in SEQ ID NO: 11;
the amino acid sequence of LCDR1 is set forth in SEQ ID NO: 17,
the amino acid sequence of LCDR2 is set forth in SEQ ID NO: 13, and
the amino acid sequence of LCDR3 is set forth in SEQ ID NO: 16.

In some embodiments of the present disclosure, provided is the anti-LAG3 antibody or the antigen-binding fragment thereof, wherein
the amino acid sequence of HCDR1 is set forth in SEQ ID NO: 9,
the amino acid sequence of HCDR2 is set forth in SEQ ID NO: 10,
the amino acid sequence of HCDR3 is set forth in SEQ ID NO: 11;
the amino acid sequence of LCDR1 is set forth in SEQ ID NO: 17,
the amino acid sequence of LCDR2 is set forth in SEQ ID NO: 15, and
the amino acid sequence of LCDR3 is set forth in SEQ ID NO: 16.

In some embodiments of the present disclosure, provided is the anti-LAG3 antibody or the antigen-binding fragment thereof, wherein
the heavy chain variable region of the anti-LAG3 antibody comprises an amino acid sequence set forth in SEQ ID NO: 2, and the light chain variable region comprises an amino acid sequence set forth in SEQ ID NO: 4;
the heavy chain variable region of the anti-LAG3 antibody comprises an amino acid sequence set forth in SEQ ID NO: 2, and the light chain variable region comprises an amino acid sequence set forth in SEQ ID NO: 6;
   or
the heavy chain variable region of the anti-LAG3 antibody comprises an amino acid sequence set forth in SEQ ID NO: 2, and the light chain variable region comprises an amino acid sequence set forth in SEQ ID NO:8.

In some embodiments of the present disclosure, provided is the anti-LAG3 antibody or the antigen-binding fragment thereof, wherein
the amino acid sequence of the heavy chain variable region of the anti-LAG3 antibody is set forth in SEQ ID NO: 2, and the amino acid sequence of the light chain variable region of the anti-LAG3 antibody is set forth in SEQ ID NO: 4;
the amino acid sequence of the heavy chain variable region of the anti-LAG3 antibody is set forth in SEQ ID NO: 2, and the amino acid sequence of the light chain variable region of the anti-LAG3 antibody is set forth in SEQ ID NO: 6;
   or
the amino acid sequence of the heavy chain variable region of the anti-LAG3 antibody is set forth in SEQ ID NO: 2, and the amino acid sequence of the light chain variable region of the anti-LAG3 antibody is set forth in SEQ ID NO: 8.

In some embodiments of the present disclosure, provided is the anti-LAG3 antibody or the antigen-binding fragment thereof, wherein the anti-LAG3 antibody or the antigen-binding fragment thereof is selected from Fab, Fab', F(ab')₂, Fd, Fv, dAb, a complementarity determining region fragment, a single chain antibody, a humanized antibody, and a chimeric antibody.

In some embodiments of the present disclosure, provided is the anti-LAG3 antibody or the antigen-binding fragment thereof, wherein the anti-LAG3 antibody comprises a non-CDR region derived from a human antibody.

In some embodiments of the present disclosure, provided is the anti-LAG3 antibody or the antigen-binding fragment thereof, wherein, the anti-LAG3 antibody comprises a constant region derived from human antibody;
preferably, the anti-LAG3 antibody comprises the constant region selected from a constant region of human IgG1, IgG2, IgG3, or IgG4.

In some embodiments of the present disclosure, provided is the anti-LAG3 antibody or the antigen-binding fragment thereof, wherein
the heavy chain constant region of anti-LAG3 antibody is Ig gamma-1 chain C region (e.g., as set forth in SEQ ID NO: 18) or Ig gamma-4 chain C region (e.g., as set forth in SEQ ID NO: 20), and the light chain constant region is Ig kappa chain C region (e.g., as set forth in SEQ ID NO: 19).

Amino acid sequence of IgG1 heavy chain constant region:









 (SEQ ID NO: 18)

In some embodiments of the present disclosure, the anti-LAG3 antibody is of human IgG1 subtype, and according to EU numbering system, the heavy chain constant region of the antibody has the following mutations:
L234A and L235A;
L234A and G237A;
L235A and G237A; or
L234A, L235A, and G237A.

In some embodiments of the present disclosure, the anti-LAG3 antibody is of human IgG4 subtype, and according to EU numbering system, the heavy chain constant region of the antibody has the following mutations:
F234A and L235A;
F234A and G237A;
L235A and G237A; or
F234A, L235A, and G237A.

In the present disclosure, letters before the position number represent amino acids before mutation, and letters after the position number represent amino acids after mutation, unless otherwise specified.

In some embodiments of the present disclosure, according to the EU numbering system, the heavy chain constant region of the immunoglobulin further has one or more mutations selected from:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A, and K320A.

In some embodiments of the present disclosure, the anti-LAG3 antibody is monoclonal antibody.

In some embodiments of the present disclosure, the anti-LAG3 antibody is immunoglobulin.

In some embodiments of the present disclosure, the anti-LAG3 antibody is the single-chain antibody.

Another aspect of the present disclosure relates to an antibody-drug conjugate (ADC), which comprises an antibody or an antigen-binding fragment thereof, and a small-molecule drug, wherein the antibody or its antigen-binding fragment is any anti-LAG3 antibody or its antigen-binding fragment as described in the present invention; preferably, the small-molecule drug is a small molecule cytotoxic drug or a cell agonist; more preferably, the small-molecule drug is a tumor chemotherapy drug, and the cell agonist is an agonist of immune cells, such as a T cell agonist or an NK cell agonist.

The chemotherapy drug can be conventional tumor chemotherapy drugs, such as an alkylating agent, an antimetabolite, an antitumor antibiotic, a plant-derived anticancer drug, a hormone, an immunomodulator, etc.

In some embodiments of the present disclosure, provided is the antibody-drug conjugate, wherein the anti-LAG3 antibody or the antigen-binding fragment thereof is linked to a small molecule drug via a linker; the linker can be a linker known to those skilled in the art, such as a hydrazone bond, a disulfide bond, or a peptide bond.

In some embodiments of the present disclosure, provided is the antibody-drug conjugate, wherein the molar ratio of the anti-LAG3 antibody or the antigen-binding fragment to the small molecule drug is 1: (2 - 4), such as 1:2, 1:3 or 1:4.

Another aspect of the present disclosure relates to an isolated nucleic acid molecule, wherein the isolated nucleic acid molecule encodes any of the anti-LAG3 antibody or the antigen-binding fragment thereof described in present disclosure.

Yet another aspect of the present disclosure relates to a recombinant vector comprising the isolated nucleic acid molecule of the present disclosure.

Yet another aspect of the present disclosure relates to a host cell comprising the isolated nucleic acid molecule of the present disclosure or the recombinant vector of the present disclosure.

Yet another aspect of the present disclosure relates to a method for preparing the anti-LAG3 antibody or the antigen-binding fragment thereof according to any one of present disclosure, comprising a step of culturing the host cell under suitable conditions and recovering the anti-LAG3 antibody or the antigen-binding fragment from a cell culture.

Another aspect of the present disclosure relates to a pharmaceutical composition, comprising any of the anti-LAG3 antibody or the antigen-binding fragment thereof described in the present disclosure, or any antibody-drug conjugate described in the present disclosure; optionally, it further comprises a pharmaceutically acceptable excipient. Another aspect of the present disclosure relates to a combination product, comprising a first product and a second product packaged separately, wherein,
the first product comprises the anti-LAG3 antibody or the antigen-binding fragment thereof or the antibody-drug conjugate according to present disclosure; and
the second product comprises at least an anti-PD-1 antibody or an anti-CD73 antibody;
preferably, the first product and the second product independently comprise one or more pharmaceutically acceptable excipients;
preferably, the combination product further comprises a package insert.

In some embodiments of the present disclosure, provided is the combination product, wherein
the molar ratio of the anti-LAG3 antibody or the antigen-binding fragment thereof to the anti-PD-1 antibody or anti-CD73 antibody is between (1:5) - (5:1), for example, 1:5, 1:4, 1:3, 1:2, 1:1, 2:1, 3:1, 4:1, or 5:1.

In some embodiments of the present disclosure, the combination product, wherein the anti-CD73 antibody is any of the anti-CD73 antibodies mentioned above.

Another aspect of the present disclosure relates to the anti-LAG3 antibody or antigen-binding fragment thereof or the antibody-drug conjugates according to any one of the embodiments of the present disclosure, which is for use in treating or preventing a tumor or an anemia;
preferably, the tumor is one or more selected from the group consisting of ovarian cancer, esophageal cancer, melanoma, hematological malignancy, glioblastoma, renal cell carcinoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.

Provided is the anti-LAG3 antibody or antigen-binding fragment thereof or the antibody-drug conjugates according to any one of the embodiments of the present disclosure, which is for use in treating or preventing a tumor or an anemia;
preferably, the tumor is one or more selected from the group consisting of ovarian cancer, esophageal cancer, melanoma, hematological malignancy, glioblastoma, renal cell carcinoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.

Another aspect of the present disclosure relates to a method for treating or preventing a tumor or an anemia, which comprises a step of administering to a subject in need thereof an effective amount of the anti-LAG3 antibody or the antigen-binding fragment thereof or any one of the antibody-drug conjugates in present disclosure according to any one of the embodiments of the present disclosure;
preferably, the tumor is one or more selected from the group consisting of ovarian cancer, esophageal cancer, melanoma, hematological malignancy, glioblastoma, renal cell carcinoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.

In one or more embodiments of the present disclosure, provided is the method for treating or preventing a tumor, wherein
the anti-LAG3 antibody is administered at a single dose of 0.1-100 mg per kg body weight, preferably 1-15 mg, 1-12 mg, 1-10 mg (e.g., 1 mg, 2 mg, 3 mg, 4 mg, 5 mg, 6 mg, 7 mg, 8 mg, 9 mg, or 10 mg), or 6-10 mg per kg body weight; or the anti-LAG3 antibody is administered to each subject at a single dose of 10-1000 mg (e.g., about 100 mg, about 150 mg, about 200 mg, about 250 mg, about 300 mg, about 350 mg, about 400 mg, about 450 mg, about 500 mg, about 600 mg, about 700 mg, about 800 mg, about 900 mg, or about 1000 mg), preferably 50-500 mg, 100-400 mg, 150-300 mg, 150-250 mg, or 200 mg;
preferably, administration is performed once every 3 days, 4 days, 5 days, 6 days, 10 days, 1 week, 2 weeks, or 3 weeks;
preferably, a route of administration is intravenous drip infusion or intravenous injection.

In some embodiments, the administration of the anti-LAG3 antibody is performed in cycles of 2 weeks (14 days) or 3 weeks (21 days), and preferably, the anti-LAG3 antibody is administered intravenously on the first day (D1) of each cycle. For example, the anti-LAG3 antibody is administered once every two weeks (q2w) or three weeks (q3w).

However, it will be appreciated that the total daily dose of the drug (e.g., the pharmaceutical composition) or the pharmaceutically active ingredient (e.g., the anti-LAG3 antibody or the antigen-binding fragment thereof) of the present disclosure must be determined by an attending physician within the scope of reliable medical judgment. For any specific patient, the specific therapeutically effective dose level is determined based on a variety of factors including the specific type and severity of the tumor being treated, the form of the specific drug formulation employed, the age, body weight, general health condition, gender, and diet of the patient, the time of administration, the route of administration, excretion rate, the duration of the treatment, other drugs used concurrently, and similar factors well known in the medical field. For example, the practice in the art involves initiating administration at dose levels below those required to achieve the desired therapeutic effect and gradually increasing the dose until the desired effect is attained.

In the present disclosure, unless otherwise defined, the scientific and technical terms used herein have the meanings generally understood by those skilled in the art. In addition, the laboratory operations of cell culture, molecular genetics, nucleic acid chemistry, and immunology used herein are the routine procedures widely used in the corresponding fields. Meanwhile, in order to better understand the present disclosure, the definitions and explanations of the related terms are provided below. As used herein, the term EC₅₀ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair with one "light" (L) chain and one "heavy" (H) chain). Antibody light chains are classified into κ and λ, light chains. Heavy chains are classified into µ, δ, γ, α, or ε. Isotypes of antibodies are defined as IgM, IgD, IgG, IgA, and IgE. In light chains and heavy chains, the variable region and constant region are linked by a "J" region of about 12 or more amino acids, and the heavy chain further comprises a "D" region of about 3 or more amino acids. Each heavy chain consists of a heavy chain variable region (VH) and a heavy chain constant region (CH). The heavy chain constant region consists of 3 domains (CH1, CH2, and CH3). Each light chain consists of a light chain variable region (VL) and a light chain constant region (CL). The light chain constant region consists of one domain CL. The constant region of the antibody can mediate the binding of immunoglobulins to host tissues or factors, including the binding of various cells of the immune system (e.g., effector cells) to the first component (C1q) of the classical complement system. The VH and VL regions can be further subdivided into hypervariable regions (called complementarity determining regions (CDRs)), between which conservative regions called framework regions (FRs) are distributed. Each VH and VL consists of 3 CDRs and 4 FRs arranged from amino terminus to carboxyl terminus in the following order: FR1, CDR1, FR2, CDR2, FR3, CDR3, and FR4. The variable regions (VH and VL) of each heavy chain/light chain pair form an antibody-binding site. The assignment of amino acids to the regions or domains is based on Bethesda M.d., Kabat Sequences of Proteins of Immunological Interest (National Institutes of Health, (1987 and 1991)), or Chothia & Lesk J. Mol. Biol., 1987; 196: 901-917; Chothia et al., Nature, 1989; 342: 878-883, or the definition of the IMGT numbering system, see the definition in Ehrenmann F, Kaas Q, Lefranc M P., IMGT/3Dstructure-DB and IMGT/DomainGapAlign: a database and a tool for immunoglobulins or antibodies, T cell receptors, MHC, IgSF and MhcSF[J]., Nucleic acids research, 2009; 38(suppl_1): D301-D307.

The term "antibody" is not limited by any specific method for producing the antibody. For example, the antibody includes a recombinant antibody, a monoclonal antibody, and a polyclonal antibody. The antibody may be antibodies of different isotypes, such as IgG (e.g., subtype IgG1, IgG2, IgG3, or IgG4), IgA1, IgA2, IgD, IgE, or IgM.

As used herein, the terms "mAb" and "monoclonal antibody" refer to an antibody or a fragment of an antibody that is derived from a group of highly homologous antibodies, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least two or more different antibodies which generally recognize different epitopes on an antigen. Monoclonal antibodies can generally be obtained using hybridoma technology first reported by Kohler et al. (Köhler G, Milstein C. Continuous cultures of fused cells secreting antibody of predefined specificity [J]. Nature, 1975; 256(5517): 495), but can also be obtained using recombinant DNA technology (see, e.g., U.S. Patent 4,816,567).

As used herein, the term "humanized antibody" refers to an antibody or an antibody fragment obtained when all or a part of CDR regions of a human immunoglobulin (receptor antibody) are replaced by the CDR regions of a non-human antibody (donor antibody), wherein the donor antibody may be a non-human (e.g., mouse, rat, or rabbit) antibody having expected specificity, affinity, or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature, 1986; 321: 522-525; Reichmann et al., Nature, 1988; 332: 323-329; Presta, Curr. Op. Struct. Biol., 1992; 2: 593-596; and Clark, Immunol. Today, 2000; 21: 397-402.

As used herein, the term "isolated" refers to obtaining by artificial means from a natural state. If a certain "isolated" substance or component is present in nature, it may be the case that a change occurs in its natural environment, or that it is isolated from the natural environment, or both. For example, a certain non-isolated polynucleotide or polypeptide naturally occurs in a certain living animal, and the same polynucleotide or polypeptide with high purity isolated from such a natural state is referred to as an isolated polynucleotide or polypeptide. The term "isolated" does not exclude the existence of artificial or synthetic substances or other impurities that do not affect the activity of the substance.

As used herein, the term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction, or transfection, such that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as λ phages or M13 phages; and animal viruses. Animal viruses that can be used as vectors include, but are not limited to, retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to, a promoter sequence, a transcription initiation sequence, an enhancer sequence, a selection element, and a reporter gene. In addition, the vector may further comprise a replication initiation site.

As used herein, the term "host cell" refers to cells to which vectors can be introduced, including, but not limited to, prokaryotic cells such as E. *coli* or *bacillus subtilis,* fungal cells such as yeast cells or *aspergillus,* insect cells such as S2 drosophila cells or Sf9, or animal cells such as fibroblasts, CHO cells, GS cells, COS cells, NSO cells, HeLa cells, BHK cells, HEK 293 cells, or human cells.

As used herein, the term "specifically bind" refers to a non-random binding reaction between two molecules, such as a reaction between an antibody and an antigen it targets. In some embodiments, an antibody that specifically binds to an antigen (or an antibody that is specific for an antigen) means that the antibody binds to the antigen with an affinity (K_{D}) of less than about 10⁻⁵ M, e.g., less than about 10⁻⁶ M, 10⁻⁷ M, 10⁻⁸ M, 10⁻⁹ M, or 10⁻¹⁰ M or less.

As used herein, the term "K_{D}" refers to a dissociation equilibrium constant for a specific antibody-antigen interaction and is used to describe the binding affinity between the antibody and the antigen. A smaller dissociation equilibrium constant indicates a stronger antibody-antigen binding and a higher affinity between the antibody and the antigen. Generally, antibodies bind to antigens (e.g., PD-1 protein) with a dissociation equilibrium constant (K_{D}) of less than about 10⁻⁵ M, such as less than about 10⁻⁶ M, 10⁻⁷ M, 10⁻⁸ M, 10⁻⁹ M or 10⁻¹⁰ M or less. K_{D} can be determined using methods known to those skilled in the art, e.g., using a Fortebio molecular interaction instrument.

As used herein, the terms "monoclonal antibody" and "mAb" have the same meaning and are used interchangeably; the terms "polyclonal antibody" and "pAb" have the same meaning and are used interchangeably. Besides, in the present disclosure, amino acids are generally represented by single-letter and three-letter abbreviations known in the art. For example, alanine can be represented by A or Ala. As used herein, the term "pharmaceutically acceptable carrier and/or excipient" refers to a carrier and/or excipient that is pharmacologically and/or physiologically compatible with the subject and the active ingredient, which is well known in the art (see, e.g., Remington's Pharmaceutical Sciences. Edited by Gennaro AR, 19th ed. Pennsylvania: Mack Publishing Company, 1995), and includes, but is not limited to, pH regulators, surfactants, adjuvants, and ionic strength enhancers. For example, the pH regulators include, but are not limited to, phosphate buffer; the surfactants include, but are not limited to, cationic, anionic, or non-ionic surfactants, such as Tween-80; the ionic strength enhancers include, but are not limited to, sodium chloride.

As used herein, the term "effective amount" refers to an amount sufficient to obtain or at least partially obtain a desired effect. For example, a prophylactically effective amount against a disease (e.g., a tumor) refers to an amount sufficient to prevent, stop, or delay the development of the disease (e.g., a tumor); a therapeutically effective amount refers to an amount sufficient to cure or at least partially stop the disease and complications thereof in patients suffering from the disease. It is undoubtedly within the ability of those skilled in the art to determine such an effective amount. For example, the amount effective for therapeutic purpose will depend on the severity of the disease to be treated, the overall state of the patient's own immune system, the general condition of the patient such as age, body weight, and gender, the route of administration, and other treatments given concurrently, etc.

As used herein, when referring to the amino acid sequence of lymphocyte-activation gene 3 (LAG3), it includes the full length of the LAG3 protein, or the extracellular fragment LAG3 ECD of LAG3, or a fragment comprising LAG3 ECD, and it also includes a fusion protein of the full length of the LAG3 protein or a fusion protein of LAG3 ECD, such as a fragment fused to an Fc protein fragment of mouse or human IgG (mFc or hFc). However, those skilled in the art will appreciate that in the amino acid sequence of the LAG3 protein, mutations or variations (including but not limited to, substitutions, deletions, and/or additions) can be naturally produced or artificially introduced without affecting biological functions thereof. Therefore, in the present disclosure, the term "LAG3" or "LAG3 protein" shall include all such sequences, including their natural or artificial variants. In addition, when describing a sequence fragment of the LAG3 protein, it also includes the corresponding sequence fragments in their natural or artificial variants. In the present disclosure, the terms "first" (e.g., first product) and "second" (e.g., second product) are used for distinguishing or clarity in expression and do not carry typical sequential meanings, unless otherwise specified.

### Beneficial Effects of the Present Disclosure

The present disclosure achieves one or more of the following effects:
(1) The anti-LAG3 antibody of the present disclosure has superior affinity and specificity.
(2) The anti-LAG3 antibody of the present disclosure can effectively block the interaction between LAG3 and MHC-II, thereby specifically relieving the immunosuppression of LAG3 on an organism.
(3) The antibody of the present disclosure can effectively treat or prevent tumors.

### BRIEF DESCRIPTION OF THE DRAWINGS

FIG. 1: Results of assays for the binding activity of H9L8(hG4WT), H9L9(hG4WT), and H9L10(hG4WT) for antigen human LAG3-mG1Fc by ELISA.
FIG. 2: Results of assays for the binding activity of H9L8(hG4WT), H9L9(hG4WT), and H9L10(hG4WT) to antigen human LAG3 on 293T-LAG3 cell surface by FACS.
FIG. 3: Results of assays for H9L8(hG4WT), H9L9(hG4WT), and H9L10(hG4WT) competing with antigen MHC II on Raji cell membrane surface for binding to human LAG3-mG1Fc by competitive flow cytometry.
FIG. 4: Results of assays for the biological activity of anti-LAG3 antibodies in promoting IFN-γ secretion by mixed lymphocyte reaction (MLR).
FIG. 5: Results of assays for the biological activity of anti-LAG3 antibodies in promoting IL-2 secretion by mixed lymphocyte reaction (MLR).
FIG. 6: Results of assays for the biological activity of anti-LAG3 antibodies in blocking the interaction between LAG3 and MHC-II.

### DETAILED DESCRIPTION

The embodiments of the present disclosure will be described in detail below with reference to the examples. Those skilled in the art will appreciate that the following examples are only for illustrating the present disclosure, and should not be construed as limitations to the scope of the present disclosure. Examples where the specific technologies or conditions are not specified are performed according to the technologies or conditions described in the publications of the art (e.g., see, Molecular Cloning: A Laboratory Manual, authored by J. Sambrook et al., and translated by Huang Peitang et al., third edition, Science Press) or according to the package insert. Reagents or instruments used are commercially available conventional products if the manufacturers thereof are not specified.

The positive control antibody, Relatlimab, has sequences referenced to the U.S. Patent Publication No. US20160326248A1, wherein for the heavy chain amino acid sequence, see SEQ ID NO: 1 of this patent publication, and for the light chain amino acid sequence, see SEQ ID NO: 2 of this patent publication. Relatlimab is an anti-LAG3 antibody.

Heavy chain amino acid sequence of Relatlimab:

QVQLQQWGAGLLKPSETLSLTCAVYGGSFSDYYWNWIRQPPGK
GLEWIGEINHRGSTNSNPSLKSRVTLSLDTSKNQFSLKLRSVTAA
DTAVYYCAFGYSDYEYNWFDPWGQGTLVTVSSASTKGPSVFPLA
PCSRSTSESTAALGCLVKDYFPEPVTVSWNSGALTSGVHTFPAVL

QSSGLYSLSSVVTVPSSSLGTKTYTCNVDHKPSNTKVDKRVESKY
GPPCPPCPAPEFLGGPSVFLFPPKPKDTLMISRTPEVTCVVVDVSQ
EDPEVQFNWYVDGVEVHNAKTKPREEQFNSTYRVVSVLTVLHQ
DWLNGKEYKCKVSNKGLPSSIEKTISKAKGQPREPQVYTLPPSQE
EMTKNQVSLTCLVKGFYPSDIAVEWESNGQPENNYKTTPPVLDS
DGSFFLYSRLTVDKSRWQEGNVFSCSVMHEALHNHYTQKSLSLS
LGK (SEQ ID NO: 23)

Light chain amino acid sequence of Relatlimab:

EIVLTQSPATLSLSPGERATLSCRASQSISSYLAWYQQKPGQAPRL
LIYDASNRATGIPARFSGSGSGTDFTLTISSLEPEDFAVYYCQQRS
NWPLTFGQGTNLEIKRTVAAPSVFIFPPSDEQLKSGTASVVCLLN
NFYPREAKVQWKVDNALQSGNSQESVTEQDSKDSTYSLSSTLTLS
KADYEKHKVYACEVTHQGLSSPVTKSFNRGEC (SEQ ID NO: 24)

Antibody 14C12H1L1(hG1TM) is an anti-PD-1 antibody prepared by Akeso Biopharma Inc.

Heavy chain amino acid sequence of 14C12H1L1(hG1TM):

EVQLVESGGGLVQPGGSLRLSCAASGFAFSSYDMSWVRQAPGKG
LDWVATISGGGRYTYYPDSVKGRFTISRDNSKNNLYLQMNSLRA
EDTALYYCANRYGEAWFAYWGQGTLVTVSSASTKGPSVFPLAPS
SKSTSGGTAALGCLVKDYFPEPVTVSWNSGALTSGVHTFPAVLQS
SGLYSLSSVVTVPSSSLGTQTYICNVNHKPSNTKVDKKVEPKSCD
KTHTCPPCPAPEAAGAPSVFLFPPKPKDTLMISRTPEVTCVVVDV
SHEDPEVKFNWYVDGVEVHNAKTKPREEQYNSTYRVVSVLTVL
HQDWLNGKEYKCKVSNKALPAPIEKTISKAKGQPREPQVYTLPP
SRDELTKNQVSLTCLVKGFYPSDIAVEWESNGQPENNYKTTPPVL
DSDGSFFLYSKLTVDKSRWQQGNVFSCSVMHEALHNHYTQKSLS
LSPGK (SEQ ID NO: 21)

Light chain amino acid sequence of 14C12H1L1(hG1TM):

DIQMTQSPSSMSASVGDRVTFTCRASQDINTYLSWFQQKPGKSPK
TLIYRANRLVSGVPSRFSGSGSGQDYTLTISSLQPEDMATYYCLQ
YDEFPLTFGAGTKLELKRTVAAPSVFIFPPSDEQLKSGTASVVCLL
NNFYPREAKVQWKVDNALQSGNSQESVTEQDSKDSTYSLSSTLTL
SKADYEKHKVYACEVTHQGLSSPVTKSFNRGEC (SEQ ID NO: 22)

Amino acid sequence of human LAG3-mG1Fc:

LQPGAEVPVVWAQEGAPAQLPCSPTIPLQDLSLLRRAGVTWQHQ
PDSGPPAAAPGHPLAPGPHPAAPSSWGPRPRRYTVLSVGPGGLRS
GRLPLQPRVQLDERGRQRGDFSLWLRPARRADAGEYRAAVHLR
DRALSCRLRLRLGQASMTASPPGSLRASDWVILNCSFSRPDRPAS
VHWFRNRGQGRVPVRESPHHHLAESFLFLPQVSPMDSGPWGCIL
TYRDGFNVSIMYNLTVLGLEPPTPLTVYAGAGSRVGLPCRLPAG
VGTRSFLTAKWTPPGGGPDLLVTGDNGDFTLRLEDVSQAQAGTY
TCHIHLQEQQLNATVTLAIITVTPKSFGSPGSLGKLLCEVTPVSGQ
ERFVWSSLDTPSQRSFSGPWLEAQEAQLLSQPWQCQLYQGERLL
GAAVYFTELSSPGAQRSGRAPGALPAGHLKLENLYFQGPRGPTIK
PCPPCKCPAPNLLGGPSVFIFPPKIKDVLMISLSPIVTCVVVDVSED
DPDVQISWFVNNVEVHTAQTQTHREDYNSTLRVVSALPIQHQDW
MSGKEFKCKVNNKDLPAPIERTISKPKGSVRAPQVYVLPPPEEEM
TKKQVTLTCMVTDFMPEDIYVEWTNNGKTELNYKNTEPVLDSD
GSYFMYSKLRVEKKNWVERNSYSCSVVHEGLHNHHTTKSFSRTP
GK (SEQ ID NO: 25)

The cell line 293T-LAG3 was constructed by Akeso Biopharma Inc. The cell line 293T-LAG3 was produced by viral infection of HEK293T cells using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11): 8463-8471), wherein the lentivirus expression vector used was plenti6.3/V5-huLAG3FL-BSD (LAG3, Genebank ID: NP_002277.4; vector plenti6.3/V5-BSD, purchased from Invitrogen, Cat. No. K5315-20).

The cell line Raji-PDL1 was constructed by Akeso Biopharma Inc. The cell line Raji-PDL1 was produced by viral infection of Raji cells using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11): 8463-8471), wherein the lentivirus expression vector used was plenti6.3/V5-PDL1 (PDL1, Genebank ID: NP_054862.1; vector plenti6.3/V5, purchased from Invitrogen, Cat. No. K5315-20).

The cell line Jurkat-NFAT-PD1-LAG3 was constructed by Akeso Biopharma Inc. The cell line Jurkat-NFAT-PD1-LAG3 was produced by viral infection of PD-1 effector cells (CPM, manufacturer: Promega, Cat. No. J112A) using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11): 8463-8471), wherein the lentivirus expression vector used was pCDH-huLAG3FL-RFP-NEO (LAG3, Genebank ID: NP_{_}002277.4; vector pCDH-CMV-MCS-EF1-RFP+Neo, purchased from Youbio, Cat. No. VT9005).

### Preparation Example 1: Design and Preparation of Anti-LAG3 Antibodies

### 1. Design of antibodies

The inventors creatively designed a series of antibody sequences based on the known LAG3 protein sequence (NCBI Reference Sequence: NP_002277.4), the three-dimensional crystal structure thereof, etc. Through extensive screening and testing, humanized monoclonal antibodies specifically binding to LAG3 were finally obtained, named H9L8, H9L9, and H9L10, respectively. The amino acid sequences of the heavy and light chain variable regions of the monoclonal antibodies and the encoding sequences thereof are as follows.

Nucleotide sequence of the heavy chain variable region H9v of H9L8 (360 bp):

CAGGTGCAGCTGCAGCAGTGGGGAGCTGGACTGCTGAAACCT
AGCGAGACACTGAGCCTGACCTGTGCTGTGTACGGCGGATCT
ATCAGCGATTACTACTGGAACTGGATCAGGCAGCCCCCTGGA
AAGGGACTGGAATGGATCGGAGAGATCAACTACAGGGGCACC
ACCAACTCCAATCCCTCTCTGAAGAGCAGGGTGACACTGAGC
CTCGACACAAGCAAGAATCAGTTCAGCCTGAAGCTGAGGTCC
GTGACCGCTGCTGATACAGCTGTGTACTACTGTGCCTTCGGCT
ACAGCGATTACGAGTACGATTGGTTCGACCCTTGGGGCCAGG
GAACACTGGTTACAGTGAGCTCC (SEQ ID NO: 1)

Amino acid sequence of the heavy chain variable region H9v of H9L8 (120 aa):

QVQLQQWGAGLLKPSETLSLTCAVYGGSISDYYWNWIRQPPGK
GLEWIGEINYRGTTNSNPSLKSRVTLSLDTSKNQFSLKLRSVTAA
DTAVYYCAFGYSDYEYDWFDPWGQGTLVTVSS (SEQ ID NO: 2)

Nucleotide sequence of the light chain variable region L8v of H9L8 (321 bp):

GAGATCGTTCTGACCCAGAGCCCAGCTACACTGAGCCTGTCT
CCTGGAGAGAGGGCTACACTGTCCTGCAGAGCTAGCCAGACC
ATCAGCAGCTACCTGGCTTGGTACCAGCAGAAGCCTGGCCAA
GCTCCAAGGCTGCTGATCTACGACGCCTCTAATAGGGCCACC

GGCATCCCTGCTAGATTCTCTGGAAGCGGCAGCGGAACCGAC
TTTACACTGACAATCAGCTCCCTGGAGCCCGAGGATTTCGCTG
TTTACTACTGTCAGCAGCGCAGCAACTGGCCCATCACATTCGG
ACAGGGCACAAATCTGGAGATCAAG (SEQ ID NO: 3)

Amino acid sequence of the light chain variable region L8v of H9L8 (107 aa):

EIVLTQSPATLSLSPGERATLSCRASQTISSYLAWYQQKPGQAPRL
LIYDASNRATGIPARFSGSGSGTDFTLTISSLEPEDFAVYYCQQRS
NWPITFGQGTNLEIK (SEQID NO: 4)

The nucleotide sequence of the heavy chain variable region H9v of H9L9 is identical to the nucleotide sequence of the heavy chain variable region H9v of H9L8, as set forth in SEQ ID NO: 1.

The amino acid sequence of the heavy chain variable region H9v of H9L9 is identical to the amino acid sequence of the heavy chain variable region H9v of H9L8, as set forth in SEQ ID NO: 2.

Nucleotide sequence of the light chain variable region L9v of H9L9 (321 bp):

GAGATCGTTCTGACCCAGAGCCCAGCTACACTGAGCCTGTCT
CCTGGAGAGAGGGCTACACTGTCCTGCAGAGCTAGCCAGACC
ATCAGCAGCTACCTGGCTTGGTACCAGCAGAAGCCTGGCCAA
GCTCCAAGGCTGCTGATCTACGACGGCTCTAATAGGGCCACC
GGCATCCCTGCTAGATTCTCTGGAAGCGGCAGCGGAACCGAC
TTTACACTGACAATCAGCTCCCTGGAGCCCGAGGATTTCGCTG
TTTACTACTGTCAGCAGCGCAGCAACTGGCCCCTCACATTCGG
ACAGGGCACAAATCTGGAGATCAAG (SEQ ID NO: 5)

Amino acid sequence of the light chain variable region L9v of H9L9 (107 bp):

EIVLTQSPATLSLSPGERATLSCRASQTISSYLAWYQQKPGQAPRL
LIYDGSNRATGIPARFSGSGSGTDFTLTISSLEPEDFAVYYCQQRS
NWPLTFGQGTNLEIK (SEQ ID NO: 6)

The nucleotide sequence of the heavy chain variable region H9v of H9L10 is identical to the nucleotide sequence of the heavy chain variable region H9v of H9L8, as set forth in SEQ ID NO: 1.

The amino acid sequence of the heavy chain variable region H9v of H9L10 is identical to the amino acid sequence of the heavy chain variable region H9v of H9L8, as set forth in SEQ ID NO: 2.

Nucleotide sequence of the light chain variable region L10v of H9L10 (321 bp):

GAGATCGTTCTGACCCAGAGCCCAGCTACACTGAGCCTGTCT
CCTGGAGAGAGGGCTACACTGTCCTGCAGAGCTAGCCAGTCC
ATCAGCAGCTACCTGGCTTGGTACCAGCAGAAGCCTGGCCAA
GCTCCAAGGCTGCTGATCTACGACGGCTCTAATAGGGCCACC
GGCATCCCTGCTAGATTCTCTGGAAGCGGCAGCGGAACCGAC
TTTACACTGACAATCAGCTCCCTGGAGCCCGAGGATTTCGCTG
TTTACTACTGTCAGCAGCGCAGCAACTGGCCCATCACATTCGG
ACAGGGCACAAATCTGGAGATCAAG (SEQ ID NO: 7)

Amino acid sequence of the light chain variable region L10v of H9L10 (107 bp):

EIVLTQSPATLSLSPGERATLSCRASQSISSYLAWYQQKPGQAPRL
LIYDGSNRATGIPARFSGSGSGTDFTLTISSLEPEDFAVYYCQQRS
NWPITFGQGTNLEIK (SEQ ID NO: 8)

The amino acid sequences of the CDRs of the antibody H9L8 are as follows (according to the IMGT numbering system):
HCDR1: GGSISDYY (SEQ ID NO: 9);
HCDR2: INYRGTT (SEQ ID NO: 10);
HCDR3: AFGYSDYEYDWFDP (SEQ ID NO: 11);
LCDR1: QTISSY (SEQ ID NO: 12);
LCDR2: DAS (SEQ ID NO: 13);
LCDR3: QQRSNWPIT (SEQ ID NO: 14).

The amino acid sequences of the CDRs of the antibody H9L9 are as follows (according to the IMGT numbering system):
HCDR1: GGSISDYY (SEQ ID NO: 9);
HCDR2: INYRGTT (SEQ ID NO: 10);
HCDR3: AFGYSDYEYDWFDP (SEQ ID NO: 11);
LCDR1: QTISSY (SEQ ID NO: 12);
LCDR2: DGS (SEQ ID NO: 15);
LCDR3: QQRSNWPLT (SEQ ID NO: 16).

The amino acid sequences of the CDRs of the antibody H9L10 are as follows (according to the IMGT numbering system):
HCDR1: GGSISDYY (SEQ ID NO: 9);
HCDR2: INYRGTT (SEQ ID NO: 10);
HCDR3: AFGYSDYEYDWFDP (SEQ ID NO: 11);
LCDR1: QSISSY (SEQ ID NO: 17);
LCDR2: DGS (SEQ ID NO: 15);
LCDR3: QQRSNWPIT (SEQ ID NO: 14).

### 2. Expression and purification of humanized antibodies H9L8(hG4WT), H9L9(hG4WT), and H9L10(hG4WT)

The heavy chain cDNA sequences (the encoding sequences of the variable regions are set forth in SEQ ID NO: 1; the constant regions were Ig gamma-4 chain C regions) of H9L8(hG4WT), H9L9(hG4WT), and H9L10(hG4WT), the light chain cDNA sequence (the encoding sequence of the variable region is set forth in SEQ ID NO: 3; the constant region was human Ig kappa chain C region) of H9L8(hG4WT), the light chain cDNA sequence (the encoding sequence of the variable region is set forth in SEQ ID NO: 5; the constant region was human Ig kappa chain C region) of H9L9(hG4WT), and the light chain cDNA sequence (the encoding sequence of the variable region is set forth in SEQ ID NO: 7; the constant region was human Ig kappa chain C region) of H9L10(hG4WT) were separately cloned into pUC57simple vectors (supplied by GenScript), and plasmids pUC57simple-H9, pUC57simple-L8, pUC57simple-L9, and pUC57simple-L10 were obtained, respectively. The plasmids pUC57simple-H9, pUC57simple-L8, pUC57simple-L9, and pUC57simple-L10 were each digested (HindIII&EcoRI). The heavy and light chains isolated by electrophoresis were separately subcloned into pcDNA3.1 vectors, and recombinant plasmids were extracted to co-transfect 293F cells. After 7 days of cell culture, the culture solution was separated by high-speed centrifugation, and the supernatant was concentrated and loaded onto a HiTrap MabSelect SuRe column. The protein was eluted in one step with an elution buffer. The target sample was isolated and buffer-exchanged into PBS.

Amino acid sequence of the heavy chain constant region of H9L8(hG4WT), H9L9(hG4WT), or H9L10(hG4WT):

ASTKGPSVFPLAPCSRSTSESTAALGCLVKDYFPEPVTVSWNSGA
LTSGVHTFPAVLQSSGLYSLSSVVTVPSSSLGTKTYTCNVDHKPSN
TKVDKRVESKYGPPCPPCPAPEFLGGPSVFLFPPKPKDTLMISRTP

EVTCVVVDVSQEDPEVQFNWYVDGVEVHNAKTKPREEQFNSTY
RVVSVLTVLHQDWLNGKEYKCKVSNKGLPSSIEKTISKAKGQPR
EPQVYTLPPSQEEMTKNQVSLTCLVKGFYPSDIAVEWESNGQPE
NNYKTTPPVLDSDGSFFLYSRLTVDKSRWQEGNVFSCSVMHEAL
HNHYTQKSLSLSLGK (SEQ ID NO: 20)

Amino acid sequence of the light chain constant region of

H9L8(hG4WT), H9L9(hG4WT), or H9L10(hG4WT):


 (SEQ ID NO: 19)

### Preparation Example 2: Preparation of Human Anti-Hen Egg Lysozyme Antibody

The sequence of the human anti-hen egg lysozyme IgG (anti-HEL, or human IgG, abbreviated as hIgG) antibody was derived from the variable region sequence of the Fab F10.6.6 sequence in the study reported by Acierno et al., entitled "Affinity maturation increases the stability and plasticity of the Fv domain of anti-protein antibodies" (Acierno et al., J Mol Biol., 2007; 374(1): 130-46). The preparation method was as follows:
Nanjing GenScript Biotech was entrusted to carry out codon optimization of amino acids and gene synthesis on heavy and light chain (complete sequence or variable region) genes of the human IgG antibody, and by referring to the standard technologies introduced in the *Molecular Cloning: A Laboratory Manual* (Third Edition) and using standard molecular cloning techniques such as PCR, enzyme digestion, DNA gel extraction, ligation transformation, colony PCR or enzyme digestion identification, the heavy and light chain genes were subcloned into the antibody heavy chain expression vector and antibody light chain expression vector of the mammalian expression system, respectively. The heavy and light chain genes of the recombinant expression vectors were further sequenced and analyzed. After the sequences were verified to be correct, a medium or large amount of endotoxin-free expression plasmids were prepared, and the heavy and light chain expression plasmids were transiently co-transfected into HEK293 cells for recombinant antibody expression. After 7 days of culture, the cell culture solutions were collected and subjected to affinity purification on a rProtein A column (GE), and the quality of the resulting antibody sample was determined using SDS-PAGE and SEC-HPLC standard analysis techniques.

### Example 1: Assays for Binding Activity of Anti-LAG3 Antibodies for Antigen by ELISA

An ELISA plate was coated with 2 µg/mL human LAG3-mG1Fc and incubated overnight at 4 °C. Then, the antigen-coated ELISA plate was washed once with PBST and blocked with a PBS solution containing 1% BSA (blocking solution) at 37 °C for 2 h. After blocking, the ELISA plate was washed 3 times with PBST. The antibodies serially diluted with a PBST solution (the dilution gradients for the antibodies are shown in Table 1) were added. The ELISA plate containing the test antibodies was incubated at 37 °C for 30 min and then washed 3 times with PBST. After washing, an HRP-labeled goat anti-human IgG (H+L) (Jackson, Cat. No. 109-035-088) secondary antibody working solution diluted at a ratio of 1:5000 was added, and the plate was then incubated at 37 °C for 30 min. After the incubation, the plate was washed 4 times with PBST. Color development was then performed with TMB (Neogen, 308177) in the dark for 5 min, and a stop solution was added to stop the color development reaction. The ELISA plate was immediately placed into a microplate reader, and the OD at a wavelength of 450 nm in each well of the ELISA plate was measured. The data were analyzed and processed using SoftMax Pro 6.2.1 software.

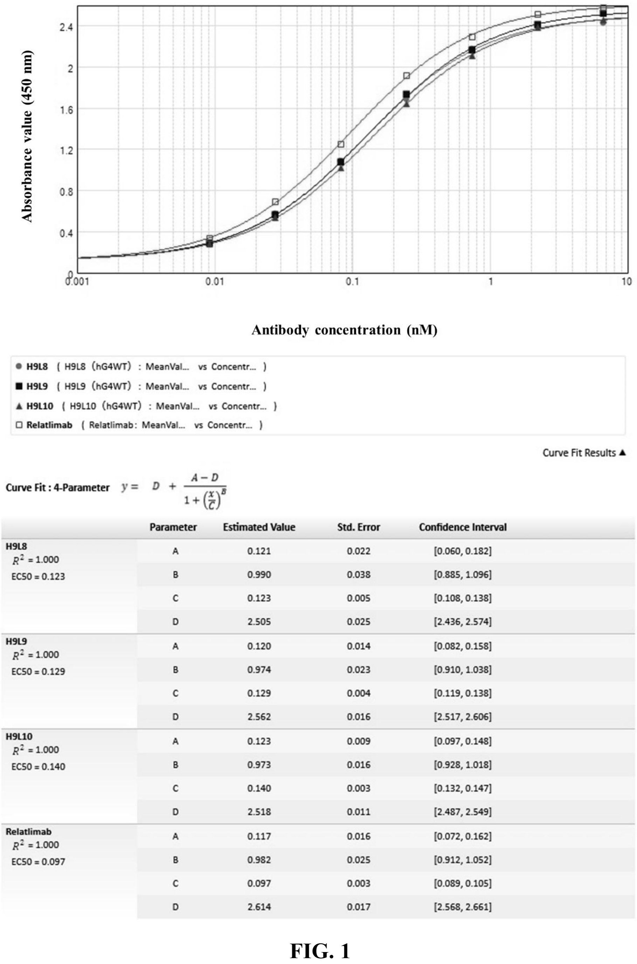

The assay results are shown in Table 1 and FIG. 1.

**Table 1. Results of assays for the binding of H9L8(hG4WT), H9L9(hG4WT), and H9L10(hG4WT) to antigen human LAG3-mG1Fc by ELISA**

| | **Human LAG3-mG1Fc, 2 µg/mL, 50 µL/well** | | | | | | | |
|---|---|---|---|---|---|---|---|---|
| **Antibody dilution (µg/mL)** | **H9L8 (hG4WT)** | | **H9L9 (hG4WT)** | | **H9L10 (hG4WT)** | | **Relatlimab** | |
| **1** | **2.403** | **2.463** | **2.485** | **2.555** | **2.438** | **2.471** | **2.568** | **2.589** |
| **0.3** | **2.414** | **2.399** | **2.420** | **2.412** | **2.345** | **2.419** | **2.468** | **2.557** |
| **0.1** | **2.201** | **2.141** | **2.200** | **2.135** | **2.121** | **2.107** | **2.260** | **2.327** |
| **0.03** | **1.734** | **1.647** | **1.783** | **1.691** | **1.637** | **1.657** | **1.868** | **1.975** |
| **0.01** | **1.104** | **1.065** | **1.089** | **1.054** | **1.019** | **1.018** | **1.220** | **1.288** |
| **0.003** | **0.592** | **0.558** | **0.567** | **0.560** | **0.525** | **0.540** | **0.649** | **0.725** |
| **0.001** | **0.282** | **0.277** | **0.298** | **0.287** | **0.275** | **0.282** | **0.324** | **0.344** |
| **0** | **0.122** | **0.123** | **0.125** | **0.118** | **0.121** | **0.126** | **0.119** | **0.121** |
| **Secondary antibody** | **Goat Anti Human IgG(H+L), HRP(1:5000)** | | | | | | | |
| **EC₅₀(nM)** | **0.123** | | **0.129** | | **0.140** | | **0.097** | |

The results show that: the antibodies H9L8(hG4WT), H9L9(hG4WT), and H9L10(hG4WT) all could effectively bind to antigen human LAG3-mG1Fc in a dose-dependent manner, and exhibited a binding activity comparable to that of the positive control antibody Relatlimab.

### Example 2: Assays for Binding Activity of Anti-LAG3 antibodies for Antigen Human LAG3 on Cell Surface by Flow Cytometry

Antibody labeling and flow cytometer detection: The 293T-LAG3 cells expressing antigen human LAG3 were digested with conventional pancreatin, and the number of cells in each collection tube was made to be 3 × 10⁵. LAG3 antibody dilutions prepared using 1% PBSA (PBS containing 1% BSA) at final concentrations of 0.0123 nM, 0.123 nM, 1.23 nM, 3.7 nM, 11.1 nM, 33.3 nM, 100 nM, and 300 nM, respectively, were each incubated with the 293T-LAG3 cells expressing LAG3 on ice for 1 h. After centrifugation and washing several times with 1% PBSA, 100 µL of FITC goat anti-human IgG (purchased from Jackson, Cat. No. 109-095-098) (diluted at a ratio of 1:300) was added to each tube, and the mixture was incubated on ice in the dark for 40 min. After washing once with 1% PBSA, 200 µL of 1% PBSA was added to resuspend the cells. Fluorescence signals were detected with FITC channel on a flow cytometer.

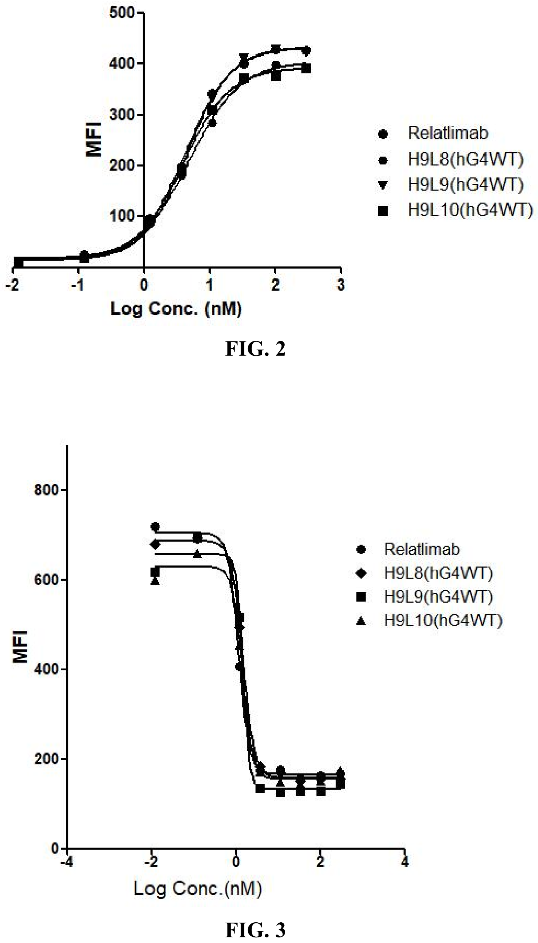

The results of the binding of humanized anti-LAG3 antibodies to 293T-LAG3 cells are shown in FIG. 2. The EC₅₀ values for the binding of the anti-LAG3 antibodies to the antigen on 293T-LAG3 cell surface are shown in Table 2.

**Table 2. Results of assays for the binding activity of anti-LAG3 antibodies for antigen on 293T-LAG3 cell surface by flow cytometer**

| **Antibody** | **EC₅₀ (nM)** |
|---|---|
| **Relatlimab** | **4.289** |
| **H9L8(hG4WT)** | **4.862** |
| **H9L9(hG4WT)** | **4.525** |
| **H9L10(hG4WT)** | **3.925** |

As can be seen from Table. 2, the anti-LAG3 antibodies could effectively bind to the target LAG3 protein on 293T-LAG3 cell surface, and the binding activity of the anti-LAG3 antibodies H9L8(hG4WT), H9L9(hG4WT), and H9L10(hG4WT) for the antigen on 293T-LAG3 cell surface was comparable to that of the positive control antibody Relatlimab.

### Example 3: Assays for Anti-LAG3 Antibodies Competing with MHC II on Raji Cell Membrane Surface for Binding to Human LAG3-mG1Fc Antigen by Competitive Flow Cytometry

Raji cells (medium: 1640 + 10% FBS) (Cell Resource Center, Shanghai Institutes for Biological Sciences, Chinese Academy of Sciences, Cat. No. TCHu 44) were added to EP tubes at 300,000 cells per sample. 1000 µL of 1% PBSA (PBS containing 1% BSA) was added. The mixture was centrifuged at 600× g for 5 min, and the supernatant was discarded. 100 µL of hIgG1 (prepared by Akeso Biopharma Inc., Batch No. 20190410) at a final concentration of 300 nM was added to each tube, and the mixture was incubated on ice for 1 h; 200 µL of 1% PBSA was added to the Raji cells after incubation, and the mixture was centrifuged at 600× g for 5 min, followed by removal of the supernatant. According to the experimental design, diluted antibodies at corresponding concentrations were added to additional clean EP tubes at 60 µL/tube. 60 µL of human LAG3-mG1Fc (prepared by Akeso Biopharma Inc., Batch No. 20190508) was added to each corresponding antibody tube, and the mixture was mixed well and pre-incubated on ice for 30 min, such that the final concentrations of the antibodies were 300 nM, 100 nM, 33.3 nM, 11.1 nM, 3.7 nM, 1.23 nM, 0.123 nM, and 0.0123 nM. The final concentration of human LAG3-mG1Fc was 3 nM. 100 µL of the pre-incubated mixture of antibody and protein was added to the cells. The resulting mixture was mixed well and incubated on ice in the dark for 1 h; 200 µL of 1% PBSA was added, and the mixture was centrifuged at 600× g for 5 min, followed by removal of the supernatant, and then the pellet was washed twice; 100 µL of an APC anti mouse antibody (purchased from Biolegend, Cat. No. 405308) (diluted at a ratio of 1:400) was added, and the mixture was mixed well and incubated on ice in the dark for 40 min; 200 µL of 1% PBSA was added, and the mixture was centrifuged at 600× g for 5 min, followed by removal of the supernatant; 200 µL of 1% PBSA was added to each tube to resuspend the cells, and then the suspension was transferred to a sample loading tube for assay on a flow cytometer.

The results are shown in FIG. 3 and Table 3. By fluorescence analysis and curve fitting, the EC₅₀ values for the competitive binding of the antibodies Relatlimab, H9L8(hG4WT), H9L9(hG4WT), and H9L10(hG4WT) were calculated to be 1.153 nM, 1.459 nM, 1.482 nM, and 1.435 nM, respectively.

**Table 3. Results of analysis for the fluorescence intensities of Relatlimab, H9L8(hG4WT), H9L9(hG4WT), and H9L10(hG4WT) in competing with MHC II on Raji cell surface for binding to the human LAG3-mG1Fc antigen by FACS**

| Antibody | EC₅₀ (nM) |
|---|---|
| Relatlimab | 1.153 |
| H9L8(hG4WT) | 1.459 |
| H9L9(hG4WT) | 1.482 |
| H9L10(hG4WT) | 1.435 |

The results show that the antibodies H9L8(hG4WT), H9L9(hG4WT), and H9L10(hG4WT) could competitively bind to LAG3 and effectively block the binding of LAG3 to MHC II on Raji cell surface in a dose-dependent manner, and exhibited an activity comparable to that of the positive control antibody Relatlimab.

### Example 4: Assays for Biological Activity of Anti-LAG3 Antibodies in Promoting IFN-γ and IL-2 Secretion by Mixed Lymphocyte Reaction (MLR)

### 1. Assays for biological activity of anti-LAG3 antibodies in promoting IFN-γ secretion in Raji-PDL1 mixed lymphocyte reaction system

Raji-PDL1 cells were conventionally subcultured. PBMCs (from healthy donors) were thawed, cultured in 10 mL of a 1640 complete medium, and stimulated with SEB (Staphylococcal enterotoxin B) (Dianotech, Cat. No. S010201) at 0.5 µg/mL for two days. The Raji-PDL1 cells were treated with MMC (Stressmarq, Cat. No. SIH-246-10MG) at a working concentration of 2 µg/mL and incubated at 37 °C in a 5% CO₂ incubator for 1 h; the PBMCs stimulated with SEB for 2 days and the Raji-PDL1 cells treated with MMC for 1 h were collected, washed twice with PBS, then resuspended in a complete medium (i.e., RPMI 1640 + 10% FBS), and counted. The PBMCs and Raji-PDL1 cells were separately added to a U-shaped 96-well plate (Corning, Model No. 3799) at 10 × 10⁴ cells/well and co-cultured. According to the experimental design, the antibodies (the final concentrations of each antibody were 300 nM, 30 nM, and 3 nM when used alone or in combination) were added and co-cultured with the cells in an incubator for 3 days; after 3 days, the cells were centrifuged at 250× g for 5 min, and the cell culture supernatant was collected and assayed for IFN-γ by ELISA.

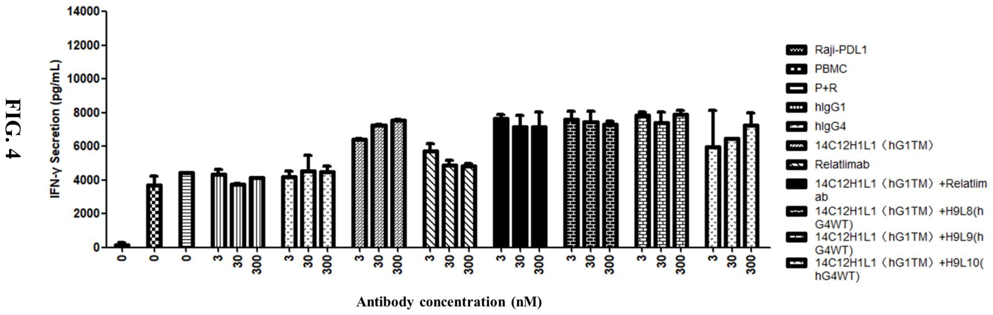

As shown in FIG. 4, the mixed culture of human PBMCs and Raji-PDL1 cells promoted the secretion of IFN-γ in PBMCs, and the addition of the antibodies to the mixed culture system could significantly induce the further secretion of IFN-γ in PBMCs. In terms of the level of activity in promoting IFN-γ secretion, the anti-LAG3 antibodies H9L8(hG4WT), H9L9(hG4WT), and H9L10(hG4WT) each in combination with 14C12H1L1(hG1TM), and the positive control antibody Relatlimab in combination with 14C12H1L1(hG1TM) all could promote IFN-γ secretion, with comparable activities.

### 2. Assays for biological activity of anti-LAG3 antibodies in promoting IL-2 secretion in Raji-PDL1 mixed lymphocyte reaction system

Raji-PDL1 cells were conventionally subcultured. PBMCs were thawed, cultured in 10 mL of a 1640 complete medium, and stimulated with SEB (Staphylococcal enterotoxin B, purchased from Dianotech, Cat. No. S010201) at 0.5 µg/mL for two days. The Raji-PDL1 cells were treated with MMC (Stressmarq, Cat. No. SIH-246-10MG) at a working concentration of 2 µg/mL and incubated at 37 °C in a 5% CO₂ incubator for 1 h. The PBMCs stimulated with SEB for 2 days and the Raji-PDL1 cells treated with MMC for 1 h were collected, washed twice with PBS, then resuspended in a complete medium (i.e., RPMI 1640 + 10% FBS), and counted. The PBMCs and Raji-PDL1 cells were separately added to a U-shaped 96-well plate (Corning, Model No. 3799) at 10×10⁴ cells/well and co-cultured. According to the experimental design, the antibodies (the final concentrations of each antibody were 300 nM, 30 nM, and 3 nM when used alone or in combination) were added and co-cultured with the cells for 3 days; after 3 days, the cells were centrifuged at 250× g for 5 min, and the cell culture supernatant was collected and assayed for IL-2 by ELISA.

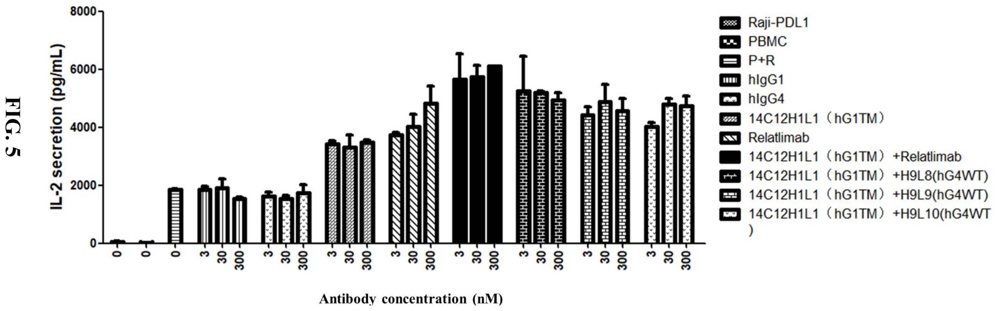

As shown in FIG. 5, the mixed culture of human PBMCs (from healthy donors) and Raji-PDL1 cells promoted the secretion of IL-2 in PBMCs to some extent, and the addition of the antibodies to the mixed culture system could significantly induce the further secretion of IL-2 in PBMCs, exhibiting a significant dose-dependent relationship. In terms of the level of activity in promoting IL-2 secretion, the anti-LAG3 antibodies H9L8(hG4WT), H9L9(hG4WT), and H9L10(hG4WT) each in combination with 14C12H1L1(hG1TM), and the positive control antibody Relatlimab in combination with 14C12H1L1(hG1TM) all could promote IL-2 secretion, with comparable activities.

### Example 5: Assays for Biological Activity Evaluation of Anti-LAG3 Antibodies in Blocking Interaction between LAG3 and MHC-II (Reporter Gene Method)

Jurkat-NFAT-PD1-LAG3 cells and Raji cells were used as a reporter gene system. After a superantigen SEE was added, a TCR-NFAT signaling pathway was activated to induce the expression of luciferase. The LAG3 on the Jurkat cells bound to the MHC-II on the Raji cells, such that the NFAT signaling pathway was inhibited, and the expression of luciferase was down-regulated. The antibody, by specifically binding to LAG3, relieved the inhibition and up-regulated the expression of luciferase.

Jurkat-NFAT-PD1-LAG3 cells and Raji cells (purchased from the Cell Resource Center, Shanghai Institutes for Biological Sciences, Chinese Academy of Sciences, Cat. No. TCHu 44) were collected and centrifuged at 110× g for 5 min, followed by removal of the supernatant. The cells were then resuspended in a 1640 + 10% FBS medium and counted. The Jurkat-NFAT-PD1-LAG3 cells were seeded into a black-bottom 96-well plate (Corning, Model No. 3916) at 10⁵ cells/well (30 µL/well); according to the experimental design, antibodies (at final concentrations of 0.3 nM, 3 nM, and 300 nM) were added at 10 µL/well, and the mixture was pre-incubated at 37 °C in a 5% CO₂ incubator for 30 min. Meanwhile, SEE (Staphylococcal Enterotoxins E, purchased from Toxin Technology, Cat. No. ET404) (at a final concentration of 0.05 ng/mL) was added to the Raji cells, and the mixture was incubated at 37 °C in a 5% CO₂ incubator for 30 min. After 30 min, the SEE-treated Raji cells were added to the 96-well plate containing Jurkat-NFAT-PD1-LAG3 cells described above at 2 × 10⁴ cells/well (40 µL/well), with the final volume of each well being 80 µL. The mixture was mixed well and incubated at 37 °C in a 5% CO₂ incubator for 6 h. After incubation, the culture plate was taken out and allowed to equilibrate to room temperature. Bright-Glo^{™}Luciferase Assay System (purchased from Promega, Cat. No. E2650) was added at 80 µL/well, and the mixture was incubated in the dark for 2 min. Then the RLU values were read. The isotype control hG4WT(hIgG4) was self-made by Akeso Biopharma Inc., with Batch No. 20190910.

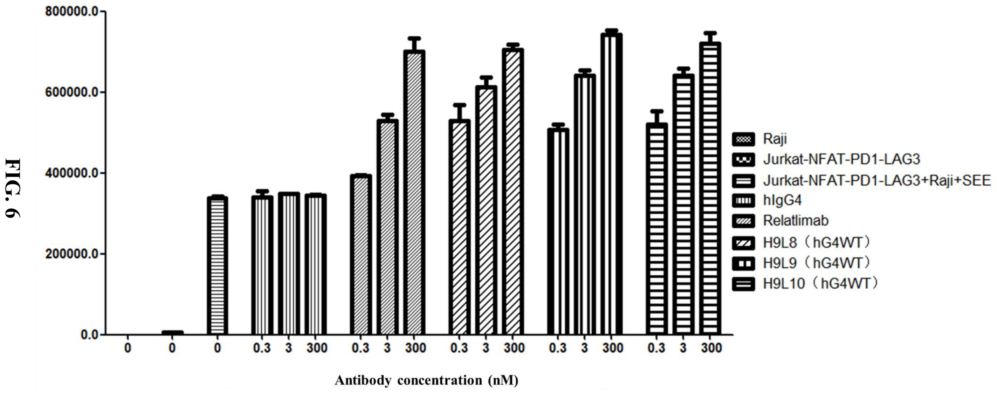

As shown in FIG. 6, the anti-LAG3 antibodies H9L8(hG4WT), H9L9(hG4WT), H9L10(hG4WT), and the positive control antibody Relatlimab all could block the interaction between LAG3 and MHC-II and thereby up-regulate the expression of luciferase, and the activities of the anti-LAG3 antibodies H9L8(hG4WT), H9L9(hG4WT), and H9L10(hG4WT) were all superior to that of the control antibody Relatlimab.

Although the specific embodiments of the present disclosure have been described in detail, those skilled in the art will appreciate that various modifications and substitutions can be made to those details according to all the teachings that have been disclosed, and these changes shall all fall within the protection scope of the present disclosure. The full scope of the present disclosure is given by the appended claims and any equivalent thereof.

## Claims

1. An anti-LAG3 antibody or an antigen-binding fragment thereof, wherein the anti-LAG3 antibody comprises a heavy chain variable region and a light chain variable region, the heavy chain variable region comprises HCDR1, HCDR2 and HCDR3, and the light chain variable region comprises LCDR1, LCDR2, LCDR3, wherein
HCDR1 comprises an amino acid sequence set forth in SEQ ID NO: 9,
HCDR2 comprises an amino acid sequence set forth in SEQ ID NO: 10,
HCDR3 comprises an amino acid sequence set forth in SEQ ID NO: 11;
LCDR1 comprises an amino acid sequence set forth in SEQ ID NO: 12 or SEQ ID NO: 17,
LCDR2 comprises an amino acid sequence set forth in SEQ ID NO: 13 or SEQ ID NO: 15,
LCDR3 comprises an amino acid sequence set forth in SEQ ID NO: 14 or SEQ ID NO: 16.

2. The anti-LAG3 antibody or the antigen-binding fragment thereof according to claim 1, wherein
LCDR1 comprises an amino acid sequence set forth in SEQ ID NO: 12,
LCDR2 comprises an amino acid sequence set forth in SEQ ID NO: 13, and
LCDR3 comprises an amino acid sequence set forth in SEQ ID NO: 14; or
LCDR1 comprises an amino acid sequence set forth in SEQ ID NO: 12,
LCDR2 comprises an amino acid sequence set forth in SEQ ID NO: 15, and
LCDR3 comprises an amino acid sequence set forth in SEQ ID NO: 16; or
LCDR1 comprises an amino acid sequence set forth in SEQ ID NO: 17,
LCDR2 comprises an amino acid sequence set forth in SEQ ID NO: 15, and
LCDR3 comprises an amino acid sequence set forth in SEQ ID NO: 14; or
LCDR1 comprises an amino acid sequence set forth in SEQ ID NO: 12,
LCDR2 comprises an amino acid sequence set forth in SEQ ID NO: 13, and
LCDR3 comprises an amino acid sequence set forth in SEQ ID NO: 16; or
LCDR1 comprises an amino acid sequence set forth in SEQ ID NO: 12,
LCDR2 comprises an amino acid sequence set forth in SEQ ID NO: 15, and
LCDR3 comprises an amino acid sequence set forth in SEQ ID NO: 14; or
LCDR1 comprises an amino acid sequence set forth in SEQ ID NO: 17,
LCDR2 comprises an amino acid sequence set forth in SEQ ID NO: 13, and
LCDR3 comprises an amino acid sequence set forth in SEQ ID NO: 14; or
LCDR1 comprises an amino acid sequence set forth in SEQ ID NO: 17,
LCDR2 comprises an amino acid sequence set forth in SEQ ID NO: 13, and
LCDR3 comprises an amino acid sequence set forth in SEQ ID NO: 16; or
LCDR1 comprises an amino acid sequence set forth in SEQ ID NO: 17,
LCDR2 comprises an amino acid sequence set forth in SEQ ID NO: 15, and
LCDR3 comprises an amino acid sequence set forth in SEQ ID NO: 16.

3. The anti-LAG3 antibody or the antigen-binding fragment thereof according to any one of claims 1 to 2, wherein
the amino acid sequence of LCDR1 is set forth in SEQ ID NO: 12,
the amino acid sequence of LCDR2 is set forth in SEQ ID NO: 13, and
the amino acid sequence of LCDR3 is set forth in SEQ ID NO: 14; or
the amino acid sequence of LCDR1 is set forth in SEQ ID NO: 12,
the amino acid sequence of LCDR2 is set forth in SEQ ID NO: 15, and
the amino acid sequence of LCDR3 is set forth in SEQ ID NO: 16; or
the amino acid sequence of LCDR1 is set forth in SEQ ID NO: 17,
the amino acid sequence of LCDR2 is set forth in SEQ ID NO: 15, and
the amino acid sequence of LCDR3 is set forth in SEQ ID NO: 14; or
the amino acid sequence of LCDR1 is set forth in SEQ ID NO: 12,
the amino acid sequence of LCDR2 is set forth in SEQ ID NO: 13, and
the amino acid sequence of LCDR3 is set forth in SEQ ID NO: 16; or
the amino acid sequence of LCDR1 is set forth in SEQ ID NO: 12,
the amino acid sequence of LCDR2 is set forth in SEQ ID NO: 15, and
the amino acid sequence of LCDR3 is set forth in SEQ ID NO: 14; or
the amino acid sequence of LCDR1 is set forth in SEQ ID NO: 17,
the amino acid sequence of LCDR2 is set forth in SEQ ID NO: 13, and
the amino acid sequence of LCDR3 is set forth in SEQ ID NO: 14; or
the amino acid sequence of LCDR1 is set forth in SEQ ID NO: 17,
the amino acid sequence of LCDR2 is set forth in SEQ ID NO: 13, and
the amino acid sequence of LCDR3 is set forth in SEQ ID NO: 16; or
the amino acid sequence of LCDR1 is set forth in SEQ ID NO: 17,
the amino acid sequence of LCDR2 is set forth in SEQ ID NO: 15, and
the amino acid sequence of LCDR3 is set forth in SEQ ID NO: 16.

4. The anti-LAG3 antibody or the antigen-binding fragment thereof according to any one of claims 1 to 3, wherein
the heavy chain variable region of the anti-LAG3 antibody comprises an amino acid sequence set forth in SEQ ID NO: 2, and the light chain variable region of the anti-LAG3 antibody comprises an amino acid sequence set forth in SEQ ID NO: 4;
the heavy chain variable region of the anti-LAG3 antibody comprises an amino acid sequence set forth in SEQ ID NO: 2, and the light chain variable region of the anti-LAG3 antibody comprises an amino acid sequence set forth in SEQ ID NO: 6; or
the heavy chain variable region of the anti-LAG3 antibody comprises an amino acid sequence set forth in SEQ ID NO: 2, and the light chain variable region of the anti-LAG3 antibody comprises an amino acid sequence set forth in SEQ ID NO: 8.

5. The anti-LAG3 antibody or the antigen-binding fragment thereof according to any one of claims 1 to 4, wherein
the amino acid sequence of the heavy chain variable region of the anti-LAG3 antibody is set forth in SEQ ID NO: 2, and the amino acid sequence of the light chain variable region of the anti-LAG3 antibody is set forth in SEQ ID NO: 4;
the amino acid sequence of the heavy chain variable region of the anti-LAG3 antibody is set forth in SEQ ID NO: 2, and the amino acid sequence of the light chain variable region of the anti-LAG3 antibody is set forth in SEQ ID NO: 6;
or
the amino acid sequence of the heavy chain variable region of the anti-LAG3 antibody is set forth in SEQ ID NO: 2, and the amino acid sequence of the light chain variable region of the anti-LAG3 antibody is set forth in SEQ ID NO: 8.

6. The anti-LAG3 antibody or the antigen-binding fragment thereof according to any one of claims 1 to 5, wherein the anti-LAG3 antibody or the antigen-binding fragment thereof is selected from Fab, Fab', F(ab')₂, Fd, Fv, dAb, a complementarity determining region fragment, a single chain antibody, a humanized antibody, and a chimeric antibody.

7. The anti-LAG3 antibody or the antigen-binding fragment thereof according to any one of claims 1 to 6, wherein the anti-LAG3 antibody comprises a non-CDR region derived from a human antibody;
preferably, the constant region of anti-LAG3 antibody is derived from a human antibody;
preferably, the constant region of the anti-LAG3 antibody is selected from a constant region of human IgG1, IgG2, IgG3, or IgG4;
the heavy chain constant region of the anti-LAG3 antibody is Ig gamma-1 chain C region (e.g., as set forth in SEQ ID NO: 18) or Ig gamma-4 chain C region (e.g., as set forth in SEQ ID NO: 20), and the light chain constant region is Ig kappa chain C region (e.g., as set forth in SEQ ID NO: 19).

8. The anti-LAG3 antibody or the antigen-binding fragment thereof according to any one of claims 1 to 7, wherein
the anti-LAG3 antibody is of human IgG1 subtype, and according to EU numbering system, the heavy chain constant region of the antibody has the following mutations:
L234A and L235A;
L234A and G237A;
L235A and G237A; or
L234A, L235A, and G237A;
or
the anti-LAG3 antibody is of human IgG4 subtype,
and according to EU numbering system, the heavy chain constant region of the antibody has the following mutations:
F234A and L235A;
F234A and G237A;
L235A and G237A; or
F234A, L235A, and G237A.

9. An antibody-drug conjugate, comprising an antibody or an antigen-binding fragment thereof and a small molecule drug, wherein the antibody or the antigen-binding fragment thereof is the antibody or the antigen-binding fragment thereof according to any one of claims 1 to 8; preferably, the small molecule drug is a small molecule cytotoxic drug or a cell agonist; more preferably, the small molecule drug is a chemotherapy drug for tumors, and the cell agonist is an agonist for immune cells, such as an agonist for T cells or NK cells.

10. The antibody-drug conjugate according to claim 9, wherein
the anti-LAG3 antibody or the antigen-binding fragment thereof is linked to the small molecule drug via a linker; for example, the linker is a hydrazone bond, a disulfide bond, or a peptide bond;
preferably, the molar ratio of the anti-LAG3 antibody or the antigen-binding fragment thereof to the small molecule drug is 1: (2 - 4).

11. An isolated nucleic acid molecule, wherein the isolated nucleic acid molecule encodes the anti-LAG3 antibody according to any one of claims 1 to 8.

12. A recombinant vector, comprising the isolated nucleic acid molecule according to claim 11.

13. A host cell, comprising the isolated nucleic acid molecule according to claim 11 or the recombinant vector according to claim 12.

14. A method for preparing the anti-LAG3 antibody or the antigen-binding fragment thereof according to any one of claims 1 to 8, comprising a step of culturing the host cell according to claim 13 under suitable conditions and recovering the anti-LAG3 antibody or the antigen-binding fragment thereof from a cell culture.

15. A pharmaceutical composition, comprising the anti-LAG3 antibody or the antigen-binding fragment thereof according to any one of claims 1 to 8 or the antibody drug conjugate according to any one of claims 9 to 10; wherein optionally, the pharmaceutical composition further comprises a pharmaceutically acceptable excipient.

16. A combination product, comprising a first product and a second product packaged separately, wherein
the first product comprises an anti-LAG3 antibody or an antigen-binding fragment thereof according to any one of claims 1 to 8 or an antibody-drug conjugate according to any one of claims 9 to 10; and
the second product comprises at least an anti-PD-1 antibody or an anti-CD73 antibody;
preferably, the first product and the second product independently comprise one or more pharmaceutically acceptable excipients;
preferably, the combination product further comprises a package insert.

17. The combination product according to claim 16, wherein, the molar ratio of anti-LAG3 antibody or the antigen-binding fragment thereof to the anti-PD-1 antibody or anti-CD73 antibody is (1:5) - (5:1).

18. Use of the anti-LAG3 antibody or the antigen-binding fragment thereof according to any one of claims 1 to 8 or the antibody-drug conjugate according to any one of claims 9 to 10 in the preparation of a medicament for treating or preventing a tumor;
preferably, the tumor is one or more selected from the group consisting of ovarian cancer, esophageal cancer, melanoma, hematological malignancy, glioblastoma, renal cell carcinoma, lung cancer, prostate cancer, bladder cancer, colon cancer, rectal cancer, liver cancer, gastric cancer, breast cancer, brain cancer, pancreatic cancer, thyroid cancer,
head and neck cancer, and kidney cancer;
preferably, the lung cancer is non-small cell lung cancer;
preferably, the hematological malignancy is leukemia;
preferably, the esophageal cancer is esophageal squamous cancer.

19. The anti-LAG3 antibody or the antigen-binding fragment thereof according to any one of claims 1 to 8 or the antibody-drug conjugate according to any one of claims 9 to 10 for use in treating or preventing a tumor;
preferably, the tumor is one or more selected from the group consisting of ovarian cancer, esophageal cancer, melanoma, hematological malignancy, glioblastoma, renal cell carcinoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.

20. A method for treating or preventing a tumor, comprising a step of administering to a subject in need thereof an effective amount of the anti-LAG3 antibody or the antigen-binding fragment thereof according to any one of claims 1 to 8 or the antibody-drug conjugate according to any one of claims 9 to 10;
preferably, the tumor is one or more selected from the group consisting of ovarian cancer, esophageal cancer, melanoma, hematological malignancy, glioblastoma, renal cell carcinoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.
